Europäisches Patentamt

(19) European Patent Office    (11) Publication number: **0 099 756**
Office européen des brevets    **B1**

(12) **EUROPEAN PATENT SPECIFICATION**

(45) Date of publication of patent specification: **01.06.88**    (51) Int. Cl.⁴: **A 61 B 5/00, G 01 N 21/31**

(21) Application number: **83304189.0**

(22) Date of filing: **19.07.83**

(54) Diaphanoscopy apparatus.

(30) Priority: **19.07.82 US 399865**

(43) Date of publication of application:
**01.02.84 Bulletin 84/05**

(45) Publication of the grant of the patent:
**01.06.88 Bulletin 88/22**

(84) Designated Contracting States:
**CH DE FR GB IT LI NL SE**

(56) References cited:
**GB-A-2 022 244**
**GB-A-2 068 537**
**US-A-3 994 591**
**US-A-4 125 858**
**US-A-4 223 995**

(73) Proprietor: **Stoller, Milton**
**68 Hilldale Road**
**West Hartford Connecticut (US)**

(72) Inventor: **Stoller, Milton**
**68 Hilldale Road**
**West Hartford Connecticut (US)**

(74) Representative: **SERJEANTS**
**25 The Crescent King Street**
**Leicester, LE1 6RX (GB)**

Courier Press, Leamington Spa, England.

## Description

### Field of Invention

The invention relates to diaphanoscopy apparatus for use for example in non-destructive testing or medical diagnostics.

### Background Art

Transillumination i.e. the passing of light through tissue, is also known as diaphanography or diaphanoscopy, and is utilised for diagnosing abnormalities. Earlier apparatus and techniques are described in the US Patent Specifications 3,127,115; 3,371,202; 3,527,932; 3,674,008; 3,711,700; 3,732,416; 3,769,963; 4,077,399; 4,212,306; 4,286,602; USSR Patent Specifications 279,879 and 591,178; in 'Diaphanologie mammaire' by C. Gross J. Radio Electrol 1972 Vol 53 No. 4pp 297—306 and 'Etude diaphanoscopique des alterations dystrophiques du sein' by C. Maggio Senologia June 1978 pp 69—71.

GB Patent Specification 2,068,537—A discloses apparatus for transilluminating biological materials with light at a selected wavelength. That wavelength can be selected by the manual movement of a holder with a purality of filters so as to place a particular filter in the path of the transilluminating light. The transilluminated light is scanned so as to build up an image in a pointwise fashion by scanning for detecting the level of transilluminated light of the selected wavelength at a plurality of positions of the transilluminated object. In one embodiment a digital memory is used for recording the levels detected and can be used to provide a colour display.

The GB Specifieaiton utilizes light in the visible and infrared region having wavelengths in totality or from 400 to about $10^6$ nanometers. The wavelength selection means disclosed employ broad or narrow band thin film optical filters with peaks at 50 nanometer intervals.

Typical embodiments can used eight different filters to give a selection of eight wavelengths or wavelength ranges which may be used in conjunction with monochromators. In use an operative must select the optimum filter for the transillumination of a particular tissue and a selection is made from the filters so as to emphasize the contrast between different types of tissue transilluminated. The successful use of the disclosed apparatus will depend on the skill of the operator and reproducibility will be similarly constrained.

As will be more apparent from the subsequent description, the invention provides an apparatus which permits transillumination to be performed in a standardized manner whilst providing sensitive displays. The invention utilizes the ratio of transilluminated light at a few different wavelengths at a particular point to provide a diaphanoscopy apparatus which is relatively easy to use and has a high degree of reliability.

It is thus amongst the aims of the invention to provide an improved diaphanoscopy apparatus.

### General Description of the Invention

The invention provides a diaphanoscopy apparatus having a means of transilluminating an object with light at a selected wavelength, a scanning means for detecting the intensity level of transilluminated light of the selected wavelength at a plurality of positions of the transilluminated object, recording means for recording the intensity levels detected for the positions and display means for representing the intensity levels detected

characterised by

control means which operate the transilluminating means to provide light pulses including light at different preselected wavelengths, the pulses being generated in a repetitive sequence;

the recording means have distinct separately addressable memory locations accessed in synchronism with the transilluminating means by the control means for recording information commensurate with the energy content of the transilluminted light at the aforementioned different pre-selected wavelengths for each of a plurality of positions of the transilluminated object;

processing means which access the recorded information for the respective wavelengths and positions to generate display correlated data as a function of the ratio of the intensity levels at the respective wavelengths at the same points; and

the display means displays the display data to represent the absorption characteristics of the object tissue. The transilluminated object may of course be human body tissue. By synchronizing the transillumination at different wavelengths with the separately addressable memory locations, it is possible to provide a diaphanoscopy apparatus which is simple. The display means may be adapted so as to produce an image of the transilluminated object. By generating the display data as a function of the ratio of the recorded energy of the respected wavelengths at the same points, a display may be obtained which is highly revealing of tissue abnormalities.

The apparatus includes a source of transillumination light at different frequencies or within different pre-selected bands of frequency. The term 'wavelengths' is hence used in a generally indicative fashion. The sequentially emitted light may share some part of the spectrum and may overlap but the spectral characteristics should be sufficiently different to give meaningful results. In accordance with one embodiment, the light source provides light in the red and near infrared regions.

In a preferred form of the invention the light which passes through the transilluminated tissue is sensed by video camera synchronized with the control means for providing light pulses. The output of the camera is thus a series of signals corresponding to images of the tissue.

The information obtained may be employed to produce a display wherein each point of the display contains information characteristic of the ratio of light at the wavelengths and a colour is assigned to each ratio so as to permit the formation of a multi-colour image. Preferably means are provided for varying the relative response to the light of different wavelengths to enable a predetermined level of display data to represent a selected type of human tissue.

### Drawings

Figure 1 is a functional block diagram of an apparatus in accordance with an embodiment of the invention;

Figure 2 is a timing diagram relating to operation of the apparatus of Figure 1;

Figure 3 is a graphical presentation for facilitating understanding of the operation of the apparatus of Figure 1.

Figure 4 is a schematic side-elevation view of a light source for use in the apparatus of Figure 1; and

Figures 5A and 5B respectively depict shutter and filter devices for use in the apparatus of Figure 4.

### Description of the preferred embodiment

With reference now to the drawings, light in the red and near infrared spectrum is employed for transilluminating human body tissue. Light at the different wavelengths chosen will be absorbed by the tissue as a function of wavelength and tissue type. "White" light is generated by a pair of strobe lights 10 and 12. The strobe lights 10 and 12 include flash lamps which generate light in a comparatively broad spectrum including the red and near infrared regions. The strobe lights 10 and 12 may include commercially available xenon flash tubes. The strobe lights 10 and 12 are respectively controlled by power supplies 14 and 16 which, in response to input commands, apply a trigger pulse to the flash tubes whereby the gas in the tube will ionize and a capacitor will discharge through the tube producing a light pulse of short duration. In accordance with one embodiment of the invention the duration of the light pulse produced by strobe lights 10 and 12 is ten microseconds and the output power of the lamps is one joule.

The light produced by strobe lights 10 and 12 is respectively collected by condensing lens systems 18 and 20 which focus the light at the ends of respective fibre optic bundles 22 and 24. Filters 26 and 28 are disposed in the light path between respective strobe lights 10 and 12 and the associated fibre optic bundles 22 and 24. The filter 26, in the embodiment being described, will pass only wavelengths in the 650 to 750 nanometer, i.e. red, region. The filter 28 will pass light only in the 750 to 850 nanometer, i.e. near infrared, region. The fibre optic bundles 22 and 24 are combined into a single bundle 30. In examining a breast, as depicted in the drawing, the free end of the fibre optic bundle 30 is placed in contact with the skin to produce a source of transilluminating light. In the manner to be described in greater detail below, the strobe lights 10 and 12 are alternatively energized in synchronism with a video system whereby the tissue of interest will be transilluminated first with red light and then with light in the near infrared region.

During transillumination video camera 32 is focused on the breast or other object being examined. In the embodiment being described camera 32 will include a silicon face plate tube which is responsive in the region from 650 nanometers to 900 nanometers. The camera tube may, for example, comprise a silicon diode array type device available from RCA under the trademark "Ultricon". The short bursts of light received by the camera 32 have the effect of discharging, in varying degrees, the surface of the video camera tube. Scanning of the tube surface by an electron beam produces, in the conventional manner, a video output signal. This analog output signal is delivered as the input to an analog-to-digital converter 34. The digitally coded signals from converter 34 are alternately supplied, via a switch 36, to a pair of frame memories 38 to 40. The memories 38 and 40 may, for example, comprise dynamic memory elements having eight bits of memory for each picture location i.e. each pixel.

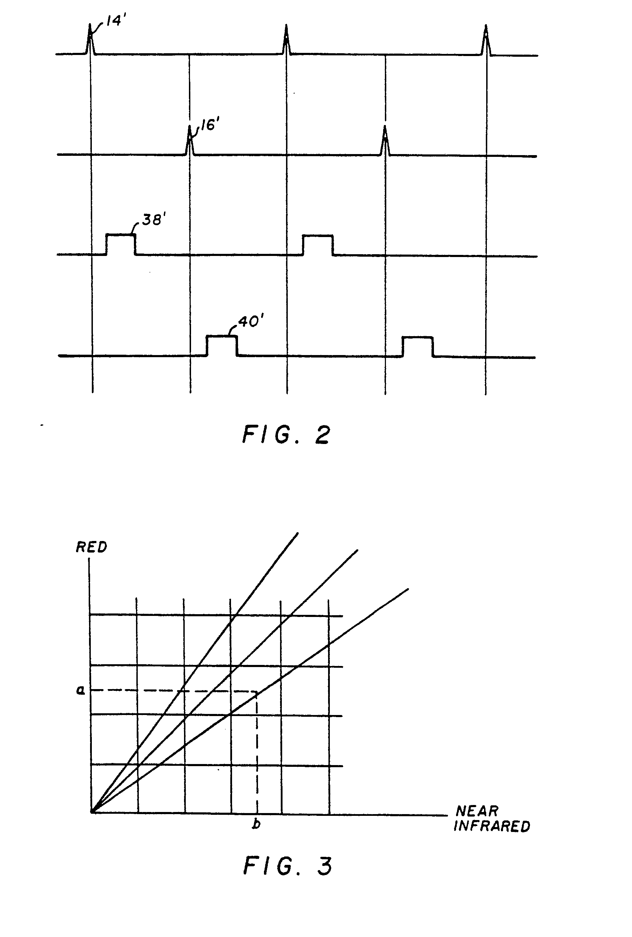

Operation of the circuitry described above is under the supervision of a controller logic 42. The controller 42 comprises a clock, down counter and gates and provides the vertical and horizontal synchronizing signals to the camera 32. Thus, referring to Figure 2, during a first vertical retrace time of the electron beam in the camera 32, the controller 42 will supply a gating pulse 14' to the power supply 14 which will cause the flash tube of strobe light 10 to fire thereby producing an intense burst of light which last a few microseconds. As discussed above, this light will be collected by the condensing lens system 18, filtered by filter 26 and the resulting "red" light delivered to the end of fibre optic bundle 30 which is in contact with the patient. The logic circuit 42 will also control the operation of switch 36, which will typically be an electronic switch, such that the digitally encoded signal produced during a single frame of scanning of the tube in camera 32 will be loaded into memory 38. The memory 38 will thus contain information commensurate with the red light which has passed through the tissue under examination. The time during which the data is being entered into memory 38, i.e. one frame time, is indicated on Figure 2 at 38'. During the next frame the vidicon is permitted to recharge fully.

During the next, i.e. third, vertical retrace time of camera 32 the controller logic 42 will produce an output signal, indicated at 16' in Figure 2, which causes the firing of the flash tube in the strobe light 12. Accordingly, the breast will, during this retrace time, be illuminated with light in the near infrared region, this light being produced from the output of the strobe light 12 by the filter 28 and delivered to the branch 24 of the bifurcated fibre optic bundle. During the next scanning of the light responsive surface of the video tube of camera 32 the control logic 42 will operate the

switch 36 so as to deliver the digitally encoded information commensurate with the near infrared light transmitted through the tissue into the frame memory 40. The time during which information is entered into the memory 40 is indicated in Figure 2 at 40'. Thus, in the embodiment being described, at the end of four frames of the scanning cycle of video camera 32, the tissue being examined will have been illuminated with light of two different colours and the information commensurate with the amount of light of each colour which has passed through the tissue will be stored in separate memories.

The information in the memories 38 and 40 is simultaneously read by an encoder which is indicated generally at 44. In actual practice, the encoder 44 will comprise a RAM which functions as a look-up table. To facilitate understanding of the disclosed embodiment of the invention, the encoder 44 has been functionally depicted as a divider 46 and a memory 48. The memory 48 will have, for example, $2^8 \times 2^8$ addresses and numbers corresponding to the intensity of two colours, typically red and green, commensurate with ratios of the numbers which may be stored at each pixel in memories 38 and 40 will be stored at the memory locations in memory 48. The data stored in the memories 38 and 40 will be read by the memory 48 twice the rate of loading of memories 38 and 40. The number stored at the corresponding memory locations in memories 38 and 40 are employed to address memory 48. The memory 48 will produce a pair of colour related, digitally coded output signals for each pixel. This is functionally equivalent to dividing the numbers stored at the memory locations in memories 38 and 40 in the divider 46 and employing the thus produced ratio to address the memory 48. The numbers which are read out of memory 48 comprise digitally coded chrominnce signals which, in the example being described, will correspond to a red "R" intensity and a green "G" intensity.

The numbers read from the memories 38 and 40 are also applied to an adder 50 where they are summed. The output of adder 50 is delivered to a divide by two circuit 52. The output of divider 52 is a digitally encoded average luminance of "Y" signal.

The "R" and "G" chrominance signals from the memory 48 are converted to analog form by means of digital-to-analog converters 54 nd 56 while the average luminance signal is converted to analog form by a digital-to-analog converter 58. The outputs of the converters 54 and 56 are respectively applied as first inputs to differential amplifiers 60 and 62. The second input to amplifiers 60 and 62 is the luminance signal from the converter 58. The combined luminance and chrominance signals appearing at the outputs of ampliiers 60 and 62 are applied to a standard TV modulator 64 which also receives synchronizing signals from the controller 42. The modulator 64 provides a composite colour video signal which is deliverd to a TV monitor 66. This composite signal will, in the customary fashion, provide horizontal sync, colour burst and colour modulation information for each frame.

It is to be noted that, in the interest of reducing the size of the look-up memory, the encoder 44 may look at the most significant six bits of the signals stored in memories 38 and 40 while the adder 50 looked at all eight bits of the stored data.

It is also be to be noted that the invention may be employed as an analytical tool wherein the ratio of the absorption by the object under examination of light at the transillumination frequencies at any point of interest may be read out and displayed on a monitor 66. To this end a joy stick 68, coupled to potentiometers indicated schematically at 70 and 72, may be employed to provide input signals to an analog-to-digital converter 74. The converter 74 will, in turn, provide address informatin to the memories 38 and 40. When there is coincidence between the addresses from converter 74 and the readout address of the memories, a spot or cursor will be displayed on monitor 66 and the contents of the memories will be delivered to a microprocessor via buffers. The microprocessor will, pursuant to its instructions, cause an alphanumeric display which may, for example, be the ratio of absorption of the two transilluminating light wavelengths.

Figure 3 may be considered a graphical representation of the function of the encoder 44. The ratio

$$\frac{a}{b}$$

of the numbers commensurate with the amount of light at the top transillumination wavelengths which is passed through the tissue under examination defines straight line curves. Each discrete curve will have assigned thereto chrominance values corresponding to red and green signal amplitude.

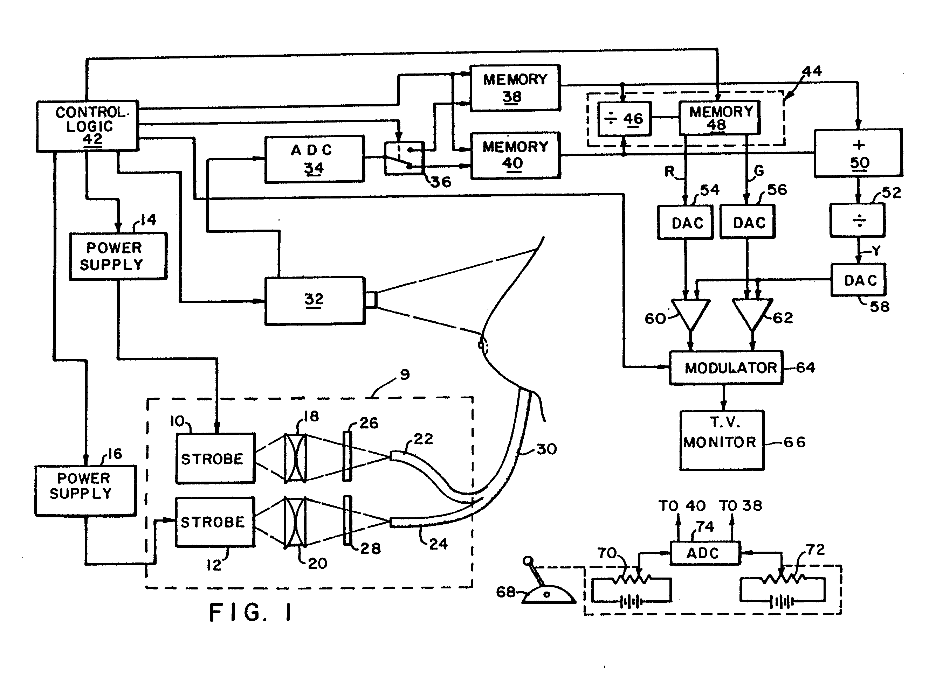

The amount of absorption of different colour light varies with the nature of the tissue being transilluminated even when tissue is normal. Accordingly, it is desirable to initially perform a "normalizing" step when using the apparatus of the invention. Since the absorption of light in the red and near infrared regions will vary as a function of whether the tissue under examination is glandular or fatty, an initial adjustment will typically be made so that the image of normal tissue will be in a pre-selected colour or colours. Thus, in a typical case, normal tissue will be displayed as a white and black image while abnormalities will be represented by colour. The colour white will be commensurate with a pre-selected a/b ratio (Figure 3) and, accordingly, the adjustment, i.e. the "normalizing", may be accomplished by varying the voltages applied to flash tubes 10 and 12 (Figure 1) to thereby vary the intensityof the two colours of light. It is also possible to separate the light passing through the tissue into two beams which are subsequently filtered and detected, the adjustment being made to the resulting signals. A further possibility would be to employ two vidicons and selectively vary the gain thereof to achieve the "normalization".

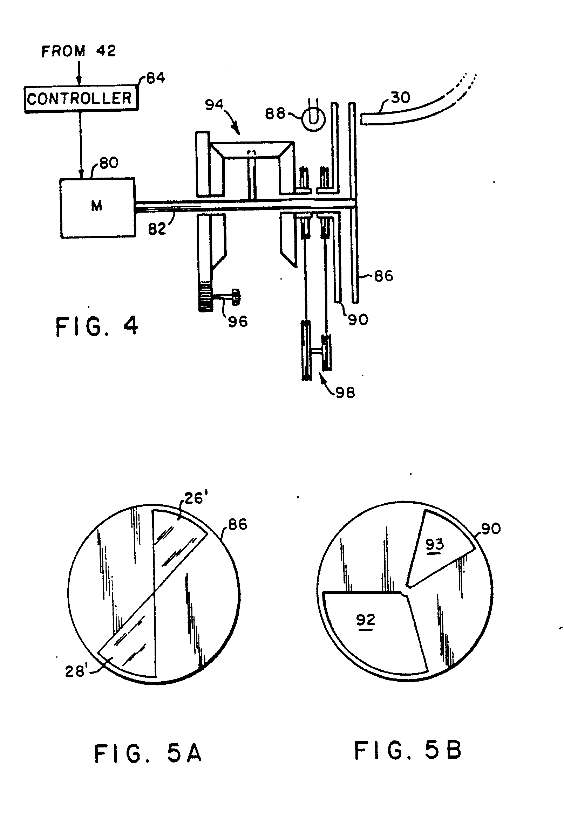

In accordance with a preferred embodiment of the invention the above-discussed step of "normalizing" is achieved through use of the apparatus of Figures 4 and 5 in plce of the pair of flash lamps 10 and 12 and their associated power supplies, lenses and filters as shown in Figure 1. In the apparatus of Figure 4, a motor 80 rotates a draft shaft 82 at a speed determined by a controller 84, controller 84 being responsibe to output pulses received from the control logic 42. A disc 86 is mounted on shaft 82 for rotation therewith. Disc 86 is provided with a pair of equal size and oppositely disposed windows. Filters, which correspond respectively to filters 26 and 28 of the Figure 1 embodiment, are respectively mounted in the windows in disc 86 as indicated at 26' and 28'. A source of white light, indicated schematically at 88, is positioned such that the light emitted therefrom will alternately pass through filters 26' and 28' as the disc 86 rotates. The end of the fibre optic bundle 30 is located in alignment with light source 88. Thus, as disc 86 is rotated the fibre optic bundle will alternately receive light in the red and near infrared regions. The speed of motor 80 will be selected such that the tissue under examination will alternately be transilluminated with the different colour light in synchronism with the operation of the camera 32, i.e. during a first frame the tissue will be illuminated with red, during a second frame the vidicon will be read out, during the third frame the vidicon screen will be recharged, during the fourth frame the tissue will be illuminated with infrared, during the fifth frame the infrared data will be read out of the vidicon, during the sixth frame the vidicon screen will be recharged, and the process will thereafter repeat.

As noted above, the "normalization" procedure will typically be performed so as to cause all normal tissue to appear in white on a black background. Thus, at the onset of each examination, the apparatus will be adjusted so that transillumination of the patient's normal tissue will result in camera 32 receiving light of equal intensity during the illumination of the tissue with both red and infrared. This normalization is achieved by mounting a shutter 90 for rotation with the disc 86. Shutter 90 is provided with a pair of different size windows 92 and 93, window 93 being substantially equal in size and shape to the filters 26' and 28'. By means of a differntial, indicated generally at 94, the differential being driven from the shaft 82, the shutter is manually rotatable relative to the disc 86 so that one of the filters 26' and 28' may be partially covered while the other remains fully uncovered. This relative rotation is achieved via a control input 94 which can be employed to either advance or retard the shutter 90 relative to the disc 86 while the two discs are rotating at the same speed. A speed reduction mechanism 98, in the form of pulleys and belts, is interposed between the differential 94 and the shutter 90 for the purpose of causing the shutter to rotate at the same speed as the filter carrying disc.

While the invention has been described as alternately illuminating the object under examination with light of two different wavelengths, the invention is not limited to transillumination with light of only two different colours or to the use of light within the frequency ranges discussed.

## Claims

1. Diaphanoscopy apparatus having a means (10, 18, 26, 22, 12, 20, 28, 24, 30; 88, 86, 90, 30) for transillumiating an object with light at a selected wavelength, a scanning means (32) for detecting the intensity level of transilluminated light of the selected wavelength at a plurality of positions of the transilluminated object, recording means (38, 40) for recording the intensity levels detected for the positions and display means (66) for representing the intensity levels detected

characterised by

control means (42) which operate the transilluminating means to provide light pulses including light at different preselected wavelengths, the pulses being generated in a repetitive sequence;

the recording means have distinct separately addressable memory locations (38) and (40) accessed in synchronism with the transilluminating means by the control means (42) for recording information commensurate with the intensity levels of the transilluminted light at the aforementioned different pre-selected wavelengths for each of a plurality of positions of the transilluminated object;

processing means (44,50) which access the recorded information for the respective wavelengths and positions to generate display data as a function of the ratio of the intensity levels at the respective wavelengths at the same points; and the display means (66) displays the display data to represent the absorption characteristics of the object tissue.

2. Diaphanoscopy apparatus according to claim 1 further characterised in that means are provided for varying the relative response to the light of different wavelengths to enable a predetermined level of display data to represent a selected type of human tissue.

3. Diaphanoscopy apparatus according to claim 1 or claim 2 further characterised in that only two wavelengths are used for transillumination, one passing wavelengths in the red region of the visible spectrum, the other passing wavelengths in the near infrared region.

4. Diaphanoscopy apparatus according to any of the preceding claims further characterised in that means (50, 52) assign intensity levels for two different colours for each display data value and modulating means (64) a connected to the display means (66) in the form of a colour television monitor to display the assigned intensity levels.

5. Diaphanoscopy apparatus according to any preceding claim further characterised in that the processing means has a means (44) for providing the ratio related display data and the display means (66) represents the ratio related data as a chrominance value and a means (50) for providing an overall light intensity related display data and the display means (66) represents the intensity related data as a luminance value.

**Patentansprüche**

1. Diaphanoskop mit einer Vorrichtung (10, 18, 26, 22, 12, 20, 28, 24, 30; 88, 86, 90, 30) zur Durchleutung eines Objektes mit Licht einer bestimmten Wellenlänge, einer Abstastvorrichtung (32) zur Ermittlung des Helligkeitspegels des Lichtes der gewählten Wellenläge nach der Durchleuchtung an einer Mehrzahl von Stellen des durchleuchteten Objektes, einer Aufnahmevorrichtung (38, 40) zur Erfassung der für die Stellen ermittelten Helligkeitspegel und einer Anzeigevorrichtung (66) zur Darstellung der ermittelten Helligkeitspegel,
gekennzeichnet durch
eine Steuervorrichtung (42) zur Bedienung der Durchleuchtungsvorrichtung derart, daß Lichtimpulse mit Licht verschiedener voreingestellter Wellenlängen erzeugt werden, wovei die Impulse in sich wiederholender Reihenfolge erzeugt werden;
die Aufnahmvorrichtung weist verschiedene, separat addressierbare Speicherzellen (38) und (40) auf, auf die die Steuervorrichtung (42) synchron mit der Durchleuchtungsvorrichtung zugreift, um dort Informationen betreffend die Helligkeitspegel des Lichtes der oben genannten verschiedenen voreingestellten Wellenlängen nach der Durchleuchtung für jeweils eine Mehrzahl von Stellen des durchleuchteten Objektes zu speichern;
eine Verarbeitungsvorrichtung (44, 50) zum Zugriff auf die für die entsprchenden Wellenlängen und Stellen gespeicherten Informationen, um Anzeigedaten als Funktion des Verhältnisses der Helligkeitspegel auf den entsprechenden Wellenlängen an den gleichen Punkten zu generieren; und
die Anzeigevorrichtung (66) die zur Darstellung der Absorptionseigenschaften des durchleuchteten Gewebes dienenden Daten anzeigt.

2. Diaphanoskop gemäß Anspruch 1, weiterhin dadurch gekennzeichnet, daß Mittel vorgesehen sind, die zur Veränderung der Relativefindlichkeit gegenüber dem Licht verschiedener Wellenlängen dienen, derart, daß mit Anzeigedaten eines vorbestimmten Niveaus ein bestimmter menschilcher Gewebetyp dargestellt werden kann.

3. Diaphanoskop gemäß Anspruch 1 oder Anspruch 2, weiterhin dadurch gegennzeichnet, daß zur Durchleuchtung nur zwei Wellenlängen verwendet werden: eine, die Wallenlängen im Rotbereich des sichtbaren Spektrums und eine, die Wellenlängen im Infrarotnahbereich durchläuft.

4. Diaphanoskop gemäß einem der vorhergehenden Ansprüche, weiterhin dadurch gekennzeichnet, daß Mittel (50, 52) jedem Anzeigedatenwert Helligkeitspegel für zwei verschiedene Farben zuweisen und eine Modulationsvorrichtung (64) an die als Farbfernseher ausgebildete Anzeigevorrichtung (66) angeschlossen ist, um die zugewiesenen Helligkeitspegel darzustellen.

5. Diaphanoskop gemäß einem der vorhergehenden Ansprüche, weiterhin dadurch gekennzeichnet, daß die Verarbeitungsvorrichtung ein Mittel (44) zur Bereitstellung der Verhältnisbezogenen Anzeigedaten, wobei die Anzeigevorrichtung (66) die Verhältnis-bezogenen Daten als Chrominanzwert darstellt, sowie ein Mittel (50 zur Bereitstellung der Helligkeitspegel-bezogenen Gesamtanzeigedaten, wobei die Anzeigevorrichtung (66) die Helligkeits-bezogenen Daten als Luminanzwert darstellt, aufweist.

**Revendications**

1. Appareil diaphanoscopique ayant des dispositifs (10, 18, 26, 22, 12, 20, 28, 24, 30, 88, 86, 90, 30) pour transilluminer un objet en utilisant de la lumière à une longueur d'onde sélectionnée, un dispositif d'exploration (32) pour déceler le niveau de lumière transilluminée de la longueur d'onde choisie pour des positions variées de l'objet transilluminé, un dispositif d'enregistrement (38, 40) pour enregistrer les niveaux d'intensité décelés pour les positions et un dispositif d'affichage (66) pour représenter les niveaux décelés caractérisés par
des dispositifs de contrôle (42) qui font opérer le dispositif de transillumination pour produire des impulsions de lumière y compris la lumière à des longueurs d'onde présélectionnées les impulsions étant produites en séquence répétitive;
les dispositifs d'enregistrement ont des cases de mémoire distinctes individuellement adressables (38) et (40) sollicitées en synchronisme avec le dispositif de transillumination par le dispositif de contrôle (42) pour enregistrer les données proportionnées aux niveaux d'intensité de la lumière transilluminée aux longueurs d'onde différentes présélectionnées ci-dessus mentionnées pour chacune des positions différentes d'un objet transilluminé;
des dispositifs de traitement (44, 50) qui sollicitent les données enregistrées pour les longueurs d'onde respectives et les positions pour produire les données d'affichage en fonction du rapport des niveaux d'intensité aux longueurs d'onde respectives aux mêmes points; et
le dispositif d'affichage (66) affiche les données d'affichage pour représenter les caractéristiques d'absorption du tissu objet.

2. L'appareil diaphanoscopique selon la revendication 1 est davantage caractérisé en ce sens qu'il possède des dispositifs pour varier la réponse relative à la lumière de différentes longueurs d'onde pour permettre un niveau prédéterminé de données d'affichage pour représenter une espèce de tissu humain sélectionnée.

3. L'appareil diaphanoscopiqe selon la revendication 1 ou la revendication 2 est davantage caractérisé en ce sens que seulement deux longueurs d'onde sont utilisées pour la transillumination, l'une passant des longueurs d'onde dans la région rouge du spectre visible, l'autre passant des longueurs d'onde dans la région presqu'infrarouge.

4. L'appareil de diaphanoscopie selon toutes les revendications précédentes est davantage caractérisé en ce sens que les dispositifs (50, 52) assignent des niveau d'intensité pour deux couleurs différentes pour chaque valeur des données d'affichage et les dispositifs de modulation (64) sont branchés sur les dispositifs d'affichage (66) sous forme de moniteur de télévision couleur pour afficher les niveaux d'intensité assignés.

5. L'appareil de diaphonoscopie selon toutes les revendications précédentes est davantage caractérisé en ce sens que le dispositif de traitement a le moyen (44) de pourvoir des données d'affichage relatives au rapport et le dispositif d'affichage (66) représente les données relatives au rapport comme valeur de chrominance et un dispositif de pourvoir des données d'affichage compréhensives relatives à l'intensité de la lumière et le dispositif d'affichage (66) représente les données relatives à l'intensité comme valeur de luminance.

FIG. I

FIG. 2

FIG. 3

0 099 756

FROM 42

CONTROLLER — 84

FIG. 4

FIG. 5A

FIG. 5B

3